# EUROPEAN PATENT APPLICATION

(11) **EP 1 990 749 A1**
(43) Date of publication of application: **12.11.2008**
(21) Application number: 07107902.4
(22) Date of filing: 10.05.2007
(51) Int. Cl.: G06F 19/00

(54) **Adaptable clinical workflow.**

(30) Priority: 08.05.2007 US 916600 P
(71) Applicant: Quadrat, 2640 Mortsel (BE)
(72) Inventor: Colaert, Dirk c/o Agfa HealthCare NV, 2640 Mortsel (BE); Chen, Helen c/o Agfa HealthCare NV, 2640 Mortsel (BE); de Roo, Joos c/o Agfa HealthCare NV, 2640 Mortsel (BE)
(74) Representative: Verbrugghe, Anne Marie L.

(57) **Abstract**

Method of generating a number of steps constituting an adaptable clinical workflow based on a recursive approach in which for each step of the workflow the following steps are executed: Selecting a number of knowledge bases to be included in a reasoning step on the basis of (a) goal(s), generating (a) query(ies) taking into account the goal(s), applying the selected knowledge bases and the generated queries to a reasoning engine so as to generate a solution, interpreting this solution by translating the solution given in a formal representation into (an) executable action(s) for the current step in the clinical workflow.

## Description

### FIELD OF THE INVENTION

The present invention relates to a method of generating individual steps of a clinical workflow.

### BACKGROUND OF THE INVENTION

Clinical pathways are considered as a means to achieve a sustainable and consistent increase in quality of care and reduction of the cost of care.

### The current problems with existing clinical pathways are:

Lack of specificity: clinical pathways are designed around a specific clinical situation but not around a specific patient. Although this gives an (intentional) consistency it makes them less useful in real life clinical situations.

Lack of flexibility: Changes in the clinical or operational context and/or in the medical knowledge is difficult to capture in running clinical pathways. In conventional IT based implementations it is very difficult if not impossible to change a running workflow instance.

Lack of merging capabilities: In a real life clinical situation patients my have multiple clinical conditions. Merging of eventual clinical pathways is not possible, using existing technologies.

Many clinical pathways rely on the local IT implementation in a hospital, such that it becomes difficult to implement pathways that span the walls of the hospital and reach to the outside world (GP, home care, ...)

Current IT based implementations use an orchestrating workflow engine which is not scalable in the context of a community healthcare setting.

Very often clinical pathways, even IT based, have no or limited connection to the run time environment. IT based clinical pathways, even if integrated in the medical record, often lack the dynamism to respond to changes in the clinical environment.

The maintenance of the existing systems is difficult due to the ever changing medical knowledge and local policies, forcing to reconsider all pathways.

It is an object of the present invention to overcome the problems listed higher.

### SUMMARY OF THE INVENTION

The present invention provides a method as set out in claim 1. Specific features for preferred embodiments of the invention are set out in the dependent claims.
Further advantages and embodiments of the present invention will become apparent from the following description.

The method of generating a number of steps constituting an adaptable clinical workflow according to the present invention comprises a recursive approach in which for each step of the workflow the following steps are executed:
- Selecting a number of knowledge bases to be included in a reasoning step on the basis of (a) goal(s),
- Generating (a) query(ies) taking into account said goal(s),
- Applying the selected knowledge bases and the generated queries to a reasoning engine so as to generate a solution,
- Interpreting said solution by translating said solution, given in a formal representation of knowledge, into executable actions for the current step in the clinical workflow.

Below some terms used in the following description and claims are defined. Preferred embodiments of the present invention are likewise explained.

### Knowledge bus:

All communication is handled on a knowledge bus being a messaging and communication system where knowledge is exchanged in a formal representation. An example of such a formal representation is RDF (resource description format).

### Knowledge base:

A knowledge base is a special kind of database for knowledge management. It provides means for the computerized collection, organization and retrieval of knowledge.
A knowledge base may use an ontology to specify its structure (entity types and relationships) and its classification scheme. An ontology together with a set of instances of its classes constitutes a knowledge base. [Wikipedia]
The knowledge bases that will be included in a reasoning step may itself be selected by reasoning.

**A Key performance indicator** (KPI) indicates a goal to be reached by the clinical workflow or by any constituent component, e.g. clinical pathways, internal processes of the IT infrastructure etc. In the context of the present invention KPI's are preferably expressed in a formal representation such as RDF.

A **query** is appropriate if it returns the necessary results to support the goals of the requesting process..

The present invention provides a method which may combine knowledge from several knowledge bases such as clinical knowledge of the patient (reflecting the latest and most up to date clinical information across the boundaries of the institution), operational knowledge from the institution or broader health care environment and general medical knowledge and reasons about it to come up with a plan to achieve certain medical goals for the patient. This plan consists of a view on the following actions that are likely to be done and a recommended next step.

Once a step is executed - or even at any time - the case can be reevaluated resulting in a new generated plan that in most cases will give a similar view on the plan and a new recommended next step Potentially however, the system will show different actions and recommendations because either the clinical context, the medical knowledge or the operational knowledge has changed. During each iteration, the recommendations and the bigger plan can be shown to the user in order to allow him to reject or approve the recommendation, or to change the sequence of actions.

The present invention uses a logic framework to implement a recursive approach to allow (1) the system to limit and/or adapt the scope of the reasoning, (2) to reason about the result of a previous reasoning and (3) to generate - just in time - the necessary conditions for optimal results, (4) rules to act on rules, which leads the invention into the world of higher order logic.

The reasoning is preferably done via a mix of deductive logic and Bayesian inference. In the Bayesian inference we conceptually separate the modeling of the medical aspects, based on medical evidences (the resolution of the Bayesian network) from the modeling of the decision strategy. The latter is done via a Bernoullian value attributed to the nodes of the network.

In the Bayesian network each node can be a hypothesis as well as evidence.

A Bernoullian value expresses the value of information and is expressed by 1-be, wherein 'be' expresses binary entropy defined as be = plogp + (1-p)log(1-p), wherein p represents probability.

A bernoullian value applied to a node of the Bayesian network will move this node in the direction of evidence or of hypothesis thereby changing the information value of that node which has direct result on the ranking of the proposed clinical workflow step.

This mechanism is used to combine medical belief with external influencing factors such as cost, preference etc. in order to come to a decision strategy.

A Bernoullian value can have values from 0 (hypothesis) to 1 (evidence).
For example when the Bernoullian is 0.5, it is a hidden node that will not be considered in decision support (i.e. not to be considered in next step, nor participate in). It will not be taken into consideration when calculating ranking. All intermediate values are possible.

An example of a **reasoning engine** that is suitable in the context of the present invention is the Euler 5 open source reasoning engine.

The present invention is generally implemented as a computer program product adapted to carry out the method of any of the claims when run on a computer and may be stored on a computer readable medium.

The workflow method of the present invention has the following advantages.

Once the knowledge is validated, it will be applied in real time clinical work by means of a software module that will bring interactive decision support capabilities into the clinical system, monitor events and raise alerts to the clinician and ultimately generates clinical workflow. The method of the present invention is such that it can be integrated in different clinical systems.

### Open System

The method of the present invention is open to other sources of knowledge, open to many clinical systems.

### Specific:

The system is able to optimize decisions for one specific patient.

### Broad Clinical Context

The architecture is suited for many different clinical contexts.

### Diverse types of knowledge

Many different types of knowledge can be used: medical, clinical, operational, infrastructural, ... to come to decisions that really make sense in a very specific situation.

Knowledge about the history (what has been done in the past) can be one of the considered sources of knowledge.

### Diverse reasoning types, mixed together

Different and appropriate reasoning technologies (for example a combination of deductive logic and Bayesian inference) will be used to optimally combine advantages of both and avoid the disadvantages

### Recursive reasoning

The system uses a recursive way of reasoning in such a way that it can reason on the results of a previous reasoning run, providing a logic framework and higher order logic.

Given clinical data and events, the system will first reason and find out what pieces of knowledge is needed to do the job. This will indicate which extra clinical data is to be taken into account and which pieces of general medical knowledge and evidences must be brought in the process. This will be the way the system will cope with the huge amount of knowledge to reason on. The iterative and recursive approach will make sure that each step is feasible and working on the right amount and kind of knowledge.

### Logic constraints

Preferably is adhered to strict monotonic ontologies, facilitating the merges of knowledge from multiple sources, and ruling out (or making very explicit) specific contexts, that otherwise would render inutile the ontologies in other contexts.

### Knowledge expression

The knowledge will preferably be expressed in an ontology format such as RDF and/or OWL. By definition these formats are mergeable. Collecting and merging distinct pieces of information, eventually authored by many different people and sources, coming from trusted parties all over the world, will be easier and more scalable than making a huge ontology or rule base with all the needed knowledge.

### Merging issues

Merging different ontologies will raise its own issues, like inconsistencies, bad sequence of procedures, overlapping procedures or prescriptions, etc ...). This will be solved by doing another run of reasoning on the merged result.

### From decision support to clinical workflow

The reasoning will not be able to only correct single actions or give advice on a single next step. It will actually be able to advice on a set of steps, advised to the clinician in a specific situation: it will generate a just in time clinical pathway. This clinical pathway can be very short (just one action) or very considerable, spanning multiple days, weeks and even years. It can also be very comprehensive e.g. spanning different departments.

After each step, or triggered by events, the reasoning may restart and eventually change the proposed steps. The result is a truly adaptable clinical workflow, be it in this project focused on infectious diseases, but very much generic and able to cope with many different situations.

All of the above, and in particular, the recursive reasoning, the logic framework and higher order logic, the reasoning for the selection of pieces of necessary knowledge and the variety of knowledge sources constitute the very innovative approach, expanding the possible clinical use of the system far beyond what exists nowadays.

## Claims

1. Method of generating a number of steps constituting an adaptable clinical workflow comprising a recursive approach in which for each step of said workflow the following steps are executed:
- Selecting a number of knowledge bases to be included in a reasoning step on the basis of (a) goal(s),
- Generating (a) query(ies) taking into account said goal(s),
- Applying the selected knowledge bases and the generated queries to a reasoning engine so as to generate a solution,
- Interpreting said solution by translating said solution given in a formal representation into (an) executable action(s) for the current step in the clinical workflow.

2. A method according to claim 1 wherein said knowledge bases, said queries, said goals are expressed in a formal representation.

3. A method according to claim 1 wherein knowledge is communicated via a knowledge bus.

4. A method according to claim 1 wherein Bayesian logic and deductive logic are combined to generate said solution.

5. A method according to claim 4 wherein a Bernoullian value is applied to a node(s) of a Bayesian network.

6. A computer program product adapted to carry out the method of any of the preceding claims when run on a computer.

7. A computer readable medium comprising computer executable program code adapted to carry out the steps of any of the preceding claims.
